# EUROPEAN PATENT APPLICATION

(11) **EP 0 674 886 A1**
(43) Date of publication of application: **04.10.1995**
(21) Application number: 95100855.6
(22) Date of filing: 23.01.1995
(51) Int. Cl.: A61F 2/36

(54) **Lightened internal articular prosthesis**

(30) Priority: 04.02.1994 IT BO940046
(71) Applicant: Società per Azioni SAMO, I-40057 Cadriano di Granarolo Emilia (Bologna) (IT)
(72) Inventor: Pipino, Francesco, I-16100 Genova (IT); Lorenzetti, Giorgio, I-40050 Quarto Inferiore (Prov. Bologna) (IT)
(74) Representative: Modiano, Guido, Dr.-Ing.

(57) **Abstract**

A lightened internal articular prosthesis (1) in which the rod (2) is constituted by a frame-like structure which is closed at the top (3) and has a frustum-shaped coupling (4) for a hip articulation ball.

## Description

The present invention relates to a lightened internal articular prosthesis.

Internal prostheses which replace damaged bone regions are known; in particular, hip prostheses are known which comprise a rod which is inserted in the femur after removing the femoral epiphysis and ends at the top either with a conical connection, for the coupling of a ball for articulating to the hip, or with the ball itself.

These conventional rods are usually solid and their installation entails forming beforehand a gauged bore in the spongy region of the bone: the insertion of these prostheses entails the destruction, removal, or damage of bone tissue and marrow.

After installation, due to the traumatic nature of the surgical operation and to the compactness of the implanted foreign structure, healing process is heavily delayed, causing considerable damage to blood circulation in the host tissues around the prosthesis.

The tissues adjacent to the prosthesis are not fed correctly and risk losing the ability to support said prosthesis.

Furthermore, conventional prostheses are very stiff, and accordingly during use bear most of the moment of flexure induced by articular forces on the assembly formed by the prosthesis and by the host bone: accordingly, the bone is loaded much less than in normal physiological conditions and therefore tends, in the course of time, to reabsorb and rarefy, losing the ability to bear loads and to support the prosthesis, causing probable disengagement.

The foregoing drawbacks make difficult to achieve longterm success of prosthesis implantation.

A principal aim of the present invention is to obviate the described drawbacks of conventional prostheses, that is to say, to provide a lightened internal articular prosthesis which does not entail excavation in the spongy bone tissue and abnormal damage to tissues and helps, by virtue of its reduced rigidity, to preserve and produce a good coupling between the prosthesis and the bone tissue.

Within the scope of this aim, another object of the present invention is to provide a structure that is simple, relatively easy to execute in practice, safe in use, effective in operation, and has a relatively low cost.

This aim and this object are achieved by the present lightened internal articular prosthesis, characterized in that the rod is constituted by a frame-like structure which is closed at the top and has a frustum-shaped coupling for a hip articulation ball.

Further characteristics will become apparent and evident from the following detailed description of a preferred but not exclusive embodiment of a lightened internal articular prostheses, according to the invention, illustrated only by way of non-limitative example in the accompanying drawings, wherein:
figure 1 is a front view of the prosthesis according to the present invention;
figure 2 is a sectional view, taken along the line II-II of figure 1;
figure 3 is a side view of the prosthesis according to the present invention;
figure 4 is a back view thereof;
figure 5 is a front view of a further embodiment of he prosthesis according to the present invention;
figure 6 is a sectional view, taken along the line VI-VI of figure 5;
figure 7 is a side view of the prosthesis of the further embodiment according to the present invention;
figure 8 is a sectional view, taken along the line VIII-VIII of figure 6;
figure 9 is a partial sectional view, taken along the line IX-IX of figure 7.

With particular reference to the above figures, the reference numeral 1 generally designates a lightened internal articular prosthesis according to the invention.

The prosthesis 1 comprises a rod 2 which is constituted by a frame-like structure that is provided, in an upward region, with a closed region 3 and a frustum-shaped coupling 4 for a hip articulation ball.

The structure of the rod 2 has, in a transverse crosssection, substantially the shape of a hollow rectangle 5 that tapers downwardly and is shaped substantially like conventional solid prostheses: in other possible embodiments, the rod 2 may have a different cross-section, for example an elliptical or other cross-section.

The frame-like structure has four longitudinal beams 6a, 6b, 6c, and 6d arranged at the edges and multiple crossmembers 7 and 8, and braces 9 which are connected to the beams and form substantially rectangular openings or slots 10 on the narrow faces 11 and triangular openings on the wide faces of the rod: the curved blendings between the beams, the cross-members, and the braces avoid the formation of dangerous concentrations of mechanical tensions in the nodes of the structure.

The number and the dimensions of the braces and of the cross-members and the materials that constitute them may be different according to the requirements to provide prostheses with modulated rigidity characteristics, in that these can be changed regularly according to the characteristics and needs of the patient. The closed region 3, which can be internally lightened by means of a contoured cavity 12, can have, at its base, a conventional collar 13 for resting on the sectioned face of the bone.

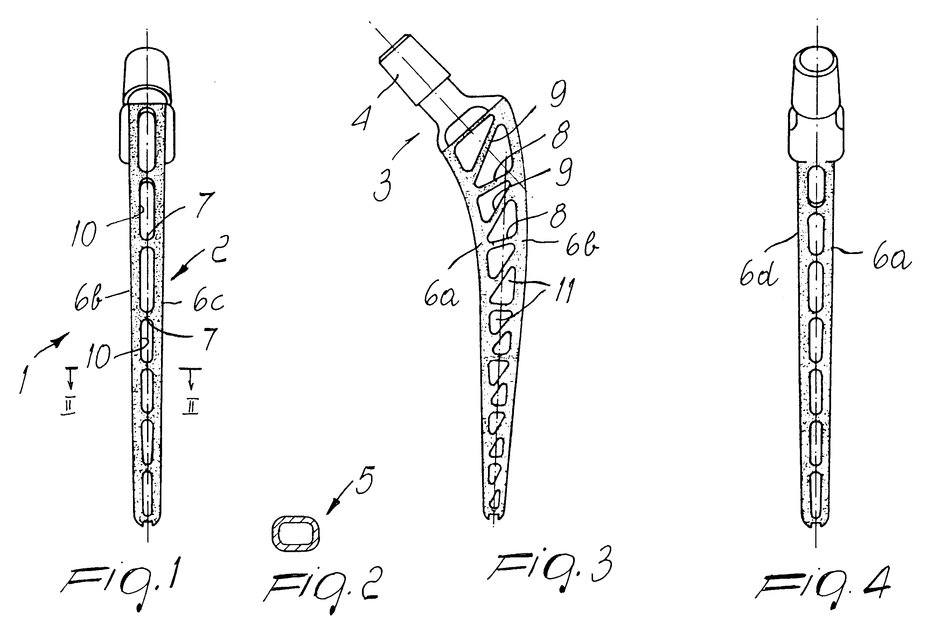

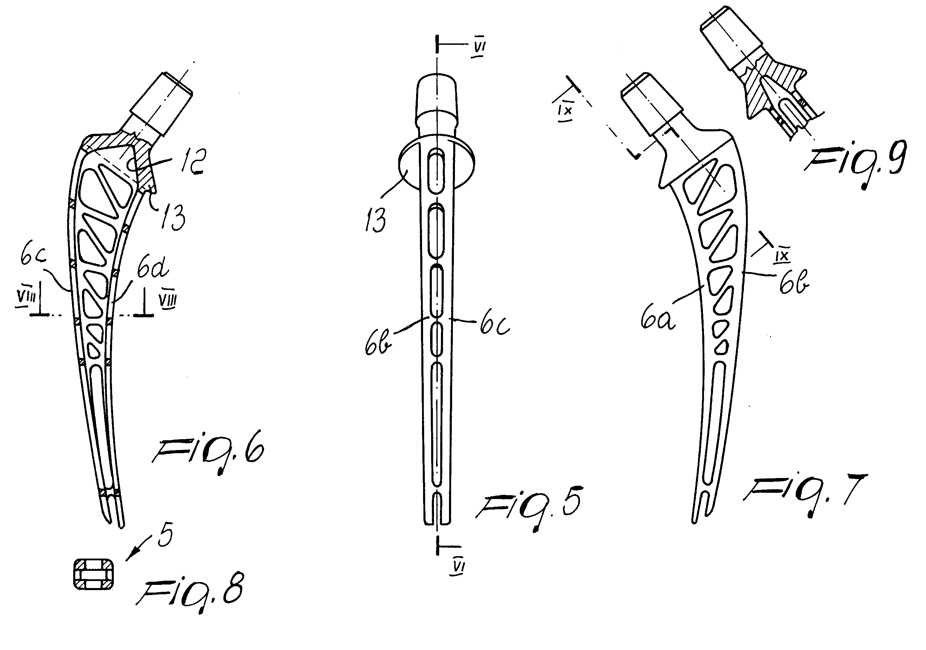

The lower end of the rod can be affected by multiple slots (figure 1) and be substantially rounded, or can be affected by a single long slot (figure 5); to facilitate insertion in the bone, the lower end of the rod may be sharp.

In a further embodiment, the lower distal end of the rod may be provided only with the axial cavity but not with the perimetric slots, to allow easy insertion in the spongy bone while avoiding the formation of a rigid coupling of the bone to the lower end of the prostheses; such a coupling would make removal of the prostheses very troublesome if another surgical operation is required.

The materials that constitute the prostheses are chosen among conventional metal alloys for permanent implants (cobalt-chrome alloys, titanium-based alloys, or the like).

It should be noted that the prosthesis according to the invention can be installed without having to form a preliminary bore in the bone and that, since the crosssection of the rod has a small area, no vascular damage is caused and precious bone matter is not removed.

With regard to the above, the rod is physically and mechanically "clamped" inside the femur; even immediately after the surgical operation, the bone trabeculae cut during insertion of the prosthesis may knit easily and rapidly.

Maximum protection of osteogenetic structures allows osteogenesis around the structures of the prosthesis.

The frame allows the massive invasion of newly produced bone in the voids provided, fully embedding the prosthetic structure.

The proliferation of the bone around the components of the prosthetic structure allows the trabecular systems of the bone to reconstitute as closely as possible to normal physiology, this being an indispensable condition for good stress distribution.

Due to its greater deformability with respect to solid prostheses and due to the fact that the rigidity of the prosthesis according to the invention can be modulated according to the characteristics of the patient, it is possible to retransfer to the femur a larger percentage of the moment of flexure, reducing one of the possible causes of failure in hip prosthesis implanting.

It has thus been observed that the invention achieves the intended aim and object.

The invention thus conceived is susceptible of numerous modifications and variations, all of which are within the scope of the inventive concept.

All the details may furthermore be replaced with other technically equivalent ones.

In practice, the materials employed, as well as the shapes and dimensions, may be any according to the requirements without thereby abandoning the protective scope of the appended claims.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. Lightened internal articular prosthesis, characterized in that the rod comprises a frame-like structure which is closed at the top (3) and has a frustum-shaped coupling (4) for a hip articulation ball.

2. Prosthesis according to claim 1, characterized in that said frame-like structure has, in transverse crosssection, substantially the shape of a hollow rectangle (5) that tapers downwardly.

3. Prosthesis according to claim 2, characterized in that said frame-like structure has four longitudinal beams (6a-6d) which are arranged at the edges and multiple crossmembers (7,8) and braces (9) which are connected to the beams and form substantially rectangular openings (10) on the narrow faces (11) of the rod and triangular openings on the wide faces of the rod.

4. Prosthesis according to claim 3, characterized in that said structure does not have perimetric openings in its lower region but only the axial longitudinal cavity.

5. Prosthesis according to claim 1, characterized in that it is provided, in an upward region, with a conventional collar (13) for resting on the sectioned face of the bone.

6. Lightened internal articular prosthesis according to one or more of the preceding claims and according to what has been described and illustrated for the specified purposes.
